# FASCICULE DE BREVET EUROPEEN

(11) **EP 2 841 935 B1**
(45) Date de publication et mention de la délivrance du brevet: **01.04.2020**
(21) Numéro de dépôt: 13721623.0
(22) Date de dépôt: 26.04.2013
(51) Int. Cl.: G01N 29/22, G01N 29/11, G01N 29/032, A61B 8/08, G01S 7/52

(54) **DISPOSITIF POUR LA MESURE D'UN PARAMETRE ULTRASONORE OU BIOMECANIQUE D'UN MILIEU VISCOELASTIQUE**
VORRICHTUNG ZUR MESSUNG EINES ULTRASCHALL- ODER BIOMECHANISCHEN PARAMETERS EINES VISKOELASTISCHEN MEDIUMS
APPARATUS FOR MEASURING AN ULTRASONIC OR BIOMECHANICAL PARAMETER OF A VISCOELASTIC ENVIRONMENT

(30) Priorité: 27.04.2012 FR 1253904
(43) Date de publication de la demande: 04.03.2015
(73) Titulaire: Echosens, 75013 Paris (FR)
(72) Inventeur: SANDRIN, Laurent, F-92240 L'hay les Roses (FR); MIETTE, Véronique, F-94800 Villejuif (FR); SASSO, Magali, F-75012 Paris (FR); OUDRY, Jennifer, F-92240 Malakoff (FR); FRADIN, Ludovic, F-75013 Paris (FR)
(74) Mandataire: Cabinet Camus Lebkiri
(86) Numéro de dépôt international: PCT/EP2013/058800
(87) Numéro de publication internationale: WO 2013/160468

(56) Documents cités:
- WO-A1-94/14375
- WO-A1-2010/063951
- WO-A1-2011/044847
- FR-A1- 2 843 290
- US-A1- 2011 282 200
- US-A1- 2012 271 166

## Description

### DOMAINE TECHNIQUE DE L'INVENTION

La présente invention concerne un dispositif pour la mesure d'un paramètre ultrasonore ou biomécanique caractéristique d'un milieu viscoélastique, comme l'élasticité ou la viscosité d'un tissu mou humain ou animal, ou plus généralement la mesure de tout paramètre d'un milieu viscoélastique présentant un signal ultrasonore rétrodiffusé après illumination ultrasonore, comme l'atténuation du signal. Elle s'applique en particulier, mais non exclusivement, à la mesure de l'élasticité de tissus adipeux d'un humain ou d'un animal.

### ETAT DE LA TECHNIQUE ANTERIEUR

On connaît un procédé pour observer la propagation d'une onde impulsionnelle de cisaillement basse fréquence simultanément en une multitude de points d'un milieu viscoélastique diffusant. A cet effet, on émet à cadence ultrarapide des ondes ultrasonores de compression qui permettent d'obtenir une succession de mesures du milieu, puis on traite en temps différé les mesures ainsi obtenues, pour déterminer les mouvements du milieu lors de la propagation de l'onde de cisaillement.

Dans les dispositifs actuels, des transducteurs ultrasonores sont utilisés pour engendrer les ondes de cisaillement basses fréquences en vibrant mécaniquement. La demande internationale WO 2011/044847 décrit un dispositif pour la mesure non destructive de l'élasticité d'un milieu viscoélastique. La demande de brevet FR2843290 décrit un dispositif pour la mesure de l'élasticité d'un organe présentant un signal ultrasonore après illumination ultrasonore, le dispositif comprenant un transducteur ultrasonore, et un actionneur électrodynamique asservi apte à faire vibrer à basse fréquence le transducteur.

Cependant un tel dispositif présente des inconvénients. En effet, il faut un opérateur pour positionner et maintenir le dispositif de façon à ce que le transducteur ultrasonore soit en regard du tissu, et différentes inclinaisons du transducteur ultrasonore par rapport à l'organe donnent différents résultats de mesure. En d'autres termes, le positionnement correct du transducteur ultrasonore dépend de l'habilité de l'opérateur.

En outre, lorsque le dispositif est appliqué sur des tissus mous, le poids du dispositif modifie les propriétés du tissu et fausse les résultats.

### DESCRIPTION GENERALE DE L'INVENTION

L'objet de l'invention a pour but de remédier aux inconvénients du dispositif de l'art antérieur décrit précédemment en fournissant un dispositif pour la mesure d'un paramètre ultrasonore ou biomécanique d'un milieu viscoélastique n'entrainant pas de modifications significatives des paramètres du milieu viscoélastique et dont les mesures ne dépendent pas de l'habilité de l'opérateur.

A cette fin, elle propose un dispositif pour la mesure d'un paramètre ultrasonore ou biomécanique d'un milieu viscoélastique, ledit dispositif comprenant au moins un transducteur ultrasonore, ledit dispositif étant caractérisé en ce qu'il comprend :
- au moins un vibreur présentant une partie fixe et une partie mobile, ledit transducteur ultrasonore étant solidaire de ladite partie mobile dudit au moins un vibreur ;
- au moins un élément adhésif solidaire du vibreur, ledit élément adhésif étant configuré pour être fixé par adhésion sur une surface en regard du milieu viscoélastique et maintenir la face d'émission et de réception du transducteur ultrasonore en regard de la surface.

De façon non limitative, on entend par surface en regard du milieu viscoélastique une surface séparant la face d'émission et de réception du transducteur ultrasonore du milieu viscoélastique,
- par exemple lorsque l'on souhaite mesurer les propriétés ultrasonores et/ou biomécaniques du tissu adipeux (le tissu adipeux étant le milieu viscoélastique dans cet exemple), l'épiderme est la surface en regard du milieu viscoélastique, ou
- par exemple lorsque l'on souhaite mesurer les propriétés ultrasonores et/ou biomécaniques de l'épiderme (l'épiderme étant le milieu viscoélastique dans cet exemple), la surface externe de l'épiderme forme la surface en regard du milieu viscoélastique.

Dans ces exemples non limitatifs, la surface en regard du tissu viscoélastique est formée par un tissu biologique de faible épaisseur.

On entend par élément adhésif, de façon non limitative, un patch, une patte adhésive, du ruban autocollant ou tout autre moyen comportant une face adhésive pouvant être fixée par collage sur un tissu biologique telle que l'épiderme ou un organe.

Grâce à l'élément adhésif (indifféremment dénommé moyens de maintien), un opérateur fixe le dispositif sur l'épiderme de manière à ce que la face d'émission et réception ultrasonore du transducteur ultrasonore soit en regard du milieu viscoélastique, et n'a plus à y toucher pendant la durée des mesures. Le vibreur est choisi de petite taille et de faible poids, comme par exemple un haut-parleur, un actionneur électrodynamique modèle réduit, ou un moteur piézo-électrique, pour que le dispositif soit maniable et léger de façon à ne pas modifier de manière significative les propriétés du milieu lorsque le dispositif est positionné en regard du milieu.

Le dispositif de l'invention permet avantageusement de réaliser une mesure d'élastographie sans prise en main du dispositif, ni même intervention de l'opérateur pendant la mesure. Ainsi, les résultats obtenus ne dépendent pas de l'habilité de l'opérateur. En d'autres termes, pour un même milieu, les mesures obtenues seront identiques et ce peu importe l'opérateur puisque ce dernier ne maintient pas le dispositif.

En outre, un tel dispositif peut par exemple être en contact direct avec un organe avant transplantation, ou un organe non protégé par les côtes de façon à déterminer comme paramètre ultrasonore, l'atténuation ultrasonore ou encore des paramètres spectraux.

Outre les caractéristiques qui viennent d'être évoquées dans le paragraphe précédent, le dispositif selon l'invention peut présenter une ou plusieurs caractéristiques complémentaires parmi les suivantes, considérées individuellement ou selon toutes les combinaisons techniquement possibles :
- le au moins un vibreur est un haut-parleur apte à vibrer,
- l'élément adhésif est fixé sur la partie fixe du vibreur et présente une extrémité libre adhésive configurée pour être fixée par adhésion sur la surface en regard du milieu viscoélastique et maintenir la face d'émission et de réception du transducteur ultrasonore en regard de la surface,
- l'extrémité libre adhésive est située sur le pourtour et à la périphérie du vibreur,
- dans une réalisation,
   - le vibreur comporte au moins deux pattes support, chacune desdites deux pattes comportant une première extrémité solidaire de la partie fixe du vibreur et une deuxième extrémité configurée pour être mise en appui sur la surface en regard du milieu viscoelastique,
   - un élément adhésif est fixé sur chacune des au moins deux pattes support et présente une extrémité libre configurée pour être fixée par adhésion sur la surface en regard du milieu viscoélastique et maintenir la face d'émission et de réception du transducteur ultrasonore en regard de la surface ;
- dans une réalisation :
   - le vibreur comporte au moins deux pattes support, chacune desdites deux pattes support comportant une première extrémité solidaire de la partie mobile du vibreur et une deuxième extrémité solidaire d'un transducteur ultrasonore, et en ce que
   - un élément adhésif est fixé sur la partie fixe du vibreur et présente une extrémité libre adhésive située à la périphérie du vibreur et configurée pour être fixée par adhésion sur la surface en regard du milieu viscoélastique et maintenir la face d'émission et de réception des transducteurs ultrasonore en regard de la surface.
- Dans une réalisation :
   - le vibreur comporte au moins deux pattes support, chacune desdites deux pattes support comportant une première extrémité solidaire de la partie fixe du vibreur et une deuxième extrémité solidaire d'un transducteur ultrasonore, et en ce que
   - un élément adhésif est fixé sur la partie fixe du vibreur et présente une extrémité libre adhésive située à la périphérie du vibreur et configurée pour être fixée par adhésion sur la surface en regard du milieu viscoélastique et maintenir la face d'émission et de réception des transducteurs ultrasonore en regard de la surface.
- ledit dispositif comporte une membrane apte à laisser passer des ultrasons sans les modifier, ladite membrane étant insérée entre la face d'émission et de réception du au moins un transducteur ultrasonore et la surface en regard du milieu viscoélastique ;
- le dispositif comporte des moyens de liaison entre l'élément adhésif et le vibreur ;
- le dispositif comporte plusieurs transducteurs ultrasonores ;
- les transducteurs sont disposés sous forme de barrette ;
- les transducteurs ultrasonores sont disposés sur un cercle dont le centre est le vibreur ;
- la fréquence centrale de vibration du vibreur (13) est comprise entre 20 et 1500 Hertz ;
- la fréquence centrale du transducteur ultrasonore est comprise entre 0,5 et 40 MegaHertz.

L'invention et ses différentes applications seront mieux comprises à la lecture de la description qui suit et à l'examen des figures qui l'accompagnent.

### BREVE DESCRIPTION DES FIGURES

Les figures ne sont présentées qu'à titre indicatif et nullement limitatif de l'invention. Les figures montrent :
- à la figure 1, une représentation schématique d'un dispositif selon un premier mode de réalisation de l'invention ;
- à la figure 2, une représentation schématique du dispositif selon un deuxième mode de réalisation de l'invention ;
- à la figure 3, une représentation schématique du dispositif selon un troisième mode de réalisation de l'invention ;
- à la figure 4, une représentation schématique du dispositif selon un quatrième mode de réalisation de l'invention ;
- à la figure 5, une représentation schématique du dispositif selon un cinquième mode de réalisation de l'invention.

### DESCRIPTION DETAILLEE D'AU MOINS UN MODE DE REALISATION DE L'INVENTION

Sauf précision contraire, un même élément apparaissant sur des figures différentes présente une référence unique.

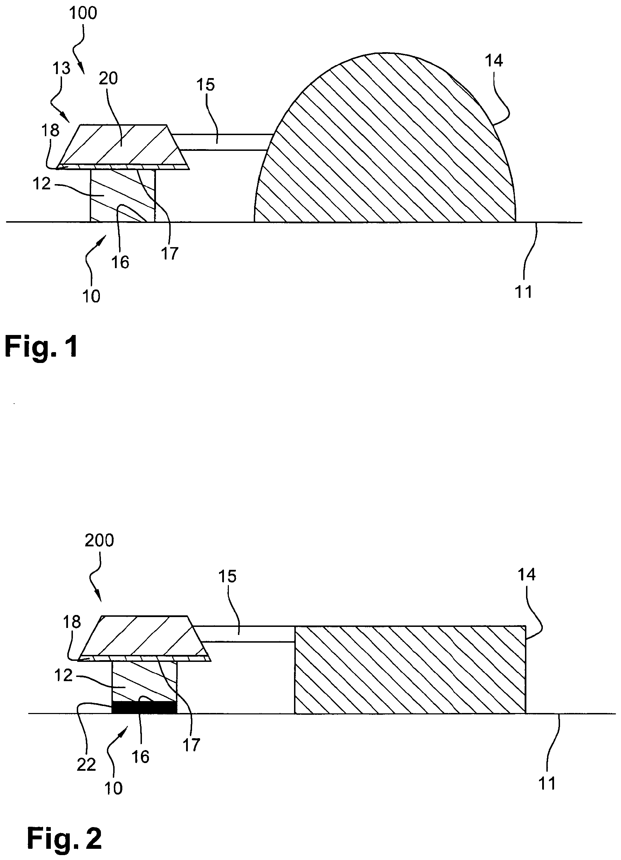

La figure 1 est une représentation schématique d'un dispositif 100 pour la mesure d'un paramètre ultrasonore ou biomécanique d'un milieu viscoélastique 10 selon un premier mode de réalisation non limitatif de l'invention, le paramètre étant corrélé à un état pathologique du milieu. Le dispositif 100 comporte :
- un transducteur ultrasonore 12, comportant une face inférieure 16 formée par la face d'émission et de réception du transducteur ultrasonore 12 et une face supérieure 17,
- un vibreur 13, qui est dans ce mode de réalisation non limitatif un haut-parleur 13 comportant une partie fixe 20 et une partie mobile 18, le vibreur 13 étant par exemple apte à réaliser des vibrations de type transitoire ou monochromatique,
- un élément adhésif 14 solidaire du vibreur 13,
- des moyens de liaison 15 entre l'élément adhésif 14 et le vibreur 13 rendant solidaire l'élément adhésif 14 de la partie fixe 20 du vibreur 13.

On note que dans d'autres modes de réalisation, le vibreur 13 est un actionneur électrodynamique modèle réduit, ou un moteur piézo-électrique, c'est-à-dire un vibreur peu coûteux, de taille réduite et léger. On entend par léger un vibreur 13 présentant par exemple un poids inférieur à 150 grammes et de préférence inférieur à 100 grammes. Dans un mode de réalisation non limitatif intéressant, le poids du vibreur est de l'ordre de 30 grammes.

La face inférieure 16 (également dénommée face d'émission et de réception 16) du transducteur repose sur une surface 11 (par exemple l'épiderme) en regard du milieu viscoélastique 10 (par exemple le tissu adipeux), et la partie mobile (formée par exemple par une membrane) 18 du haut-parleur 13 est fixée à la face supérieure 17 du transducteur 12. L'élément adhésif 14 adhère à la surface 11 (l'épiderme dans l'exemple) en regard du milieu viscoélastique 10 (le tissu adipeux dans l'exemple), et est solidaire de la partie fixe 20 du haut-parleur 13 via les moyens de liaison 15. Ainsi, l'ensemble transducteur et vibreur est solidaire de l'épiderme. Les moyens de liaison 15 sont formés par exemple par une tige 15 rigide ou flexible dont une extrémité est fixée à l'élément adhésif 14, et une autre extrémité est fixée au corps 20 du haut-parleur 13. De façon non limitative, la tige 15 s'étend selon un axe sensiblement parallèle à la surface 11 du milieu viscoélastique 10.

Grâce à l'élément adhésif 14 et aux moyens de liaison 15, l'ensemble haut-parleur 13 / transducteur 12 est maintenu dans une position stable vis-à-vis du milieu viscoélastique 10, telle que la face inférieure 16 du transducteur 12 soit au contact de la surface 11 en regard du milieu viscoélastique 10. Il est entendu qu'un gel ou une membrane de protection du transducteur ultrasonore laissant passer les ultrasons peut être positionné entre la face inférieure 16 du transducteur 12 et la surface 11.

Le haut-parleur 13 est réglé de sorte que sa partie mobile 18 vibre à basse fréquence à une fréquence centrale f1 définie. f1 est avantageusement choisie entre 20 et 1500 Hertz, et plus particulièrement entre 70 et 100 Hz. Etant fixé au transducteur 12, la membrane entraine le transducteur 12 en vibration basse fréquence, qui lui-même génère un coup basse fréquence reçu par le milieu viscoélastique. Un coup (ou vibration) reçu par le milieu viscoélastique 10 engendre la propagation d'une onde de cisaillement basse fréquence, qui se propage dans le milieu viscoélastique 10. La vitesse de déplacement de l'onde de cisaillement dépend de l'élasticité et de la viscosité du milieu viscoélastique 10.

Il convient de noter que le haut-parleur 13 peut générer un unique coup basse fréquence ou une pluralité de coups basse fréquence successifs.

Par ailleurs, le transducteur ultrasonore 12 est apte à générer des ondes ultrasonores hautes fréquences f2. La fréquence centrale f2 du transducteur 12 est avantageusement comprise entre 0,5 et 40 Megahertz, par exemple 3,5 Megahertz. Cette fréquence centrale f2 est choisie en fonction de la profondeur de pénétration souhaitée des ondes ultrasonores dans le milieu viscoélastique 10 : plus la fréquence est élevée, moins les ondes pénètrent dans le milieu viscoélastique 10. Par exemple, à 12 Megahertz, les mesures sont réalisées sur une profondeur de l'ordre de 5 mm sous l'épiderme. Les ondes ultrasonores sont réfléchies par les particules du milieu, et le signal retour est reçu par le même transducteur 12.

La partie mobile 18 du haut-parleur 13 est fixée à la face supérieure 17 du transducteur 12, par exemple par collage. Avantageusement, une fixation par collage est simple et peu coûteuse.

Le procédé utilisé par la suite pour mesurer l'élasticité du milieu viscoélastique extrait des signaux ultrasonores ou des images ultrasonores est bien connu par l'homme du métier, et est par exemple détaillé dans la demande de brevet FR2843290.

Dans un second mode de réalisation illustré sur la figure 2 d'un dispositif 200 selon l'invention, une membrane 22 est insérée entre la surface 11 en regard du milieu viscoélastique 10 et la face d'émission et de réception 16 du transducteur 12. La face d'émission et de réception 16 du transducteur repose sur la membrane 22. La membrane 22 est constituée d'un matériau laissant passer les ultrasons sans les modifier. Grâce à cette membrane 22, le dispositif 200 est réutilisable, seule la membrane 22 doit être remplacée entre deux utilisations. Cette particularité permet de respecter les conditions d'hygiène imposées dans les hôpitaux. Il est en effet facile et peu coûteux de remplacer uniquement la membrane 22.

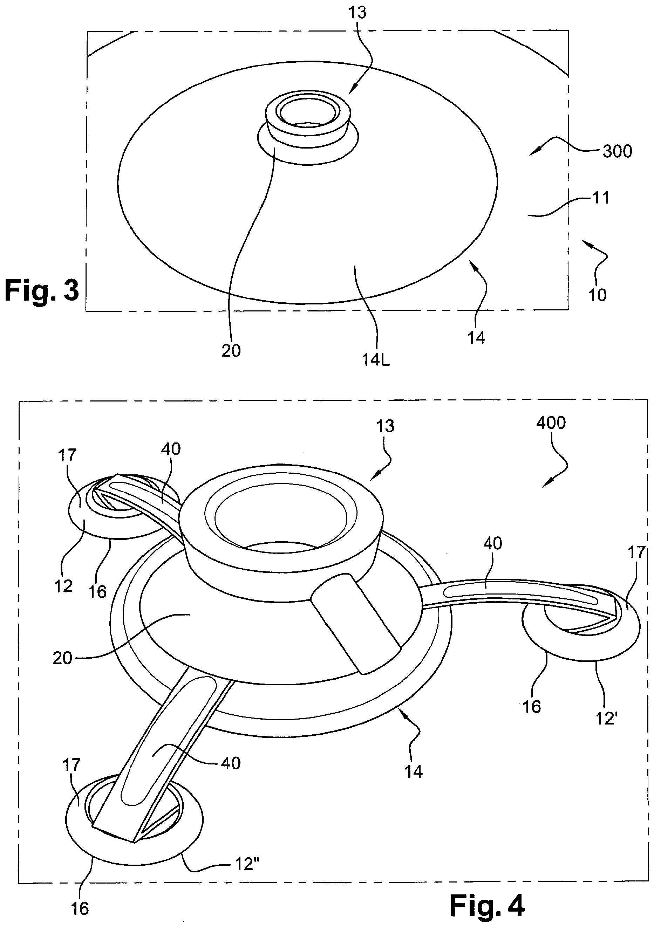

La figure 3 est une représentation schématique d'un dispositif 300 pour la mesure de l'élasticité du milieu viscoélastique 10 selon un troisième mode de réalisation non limitatif de l'invention. Le dispositif 300 comporte notamment :
- un transducteur ultrasonore 12 (non visible sur la figure), comportant une face inférieure 16 et une face supérieure 17,
- un haut-parleur 13,
- un élément adhésif 14 fixé sur la partie fixe 20 du vibreur 13 et présentant une extrémité libre 14L adhésive configurée pour être fixée par adhésion sur la surface 11 en regard du milieu viscoélastique 10 et maintenir la face d'émission et de réception du transducteur ultrasonore (non illustré) en regard de la surface 11, l'extrémité libre 14L étant formée dans cet exemple par le bord périphérique de l'élément adhésif 14.

Il convient de noter que dans cette réalisation non limitative l'extrémité libre 14L adhésive est située sur le pourtour du vibreur 13, autrement dit au niveau de toute la périphérie du vibreur 13. Dans l'exemple illustré, la partie fixe 20 du vibreur est circulaire et l'élément adhésif 14 est circulaire. On peut évidemment imaginer une autre forme de l'élément adhésif, il pourrait être rectangulaire, triangulaire, ou autre.

Grâce au faible encombrement du dispositif 300 selon l'invention, il est possible de positionner plusieurs dispositifs 300 sur la surface 11 en regard du milieu viscoélastique 10 pour effectuer des mesures simultanées.

La figure 4 est une représentation schématique d'un dispositif 400 pour la mesure de l'élasticité du milieu viscoélastique 10 selon un quatrième mode de réalisation non limitatif de l'invention. Le dispositif 400 comporte :
- quatre transducteurs ultrasonores 12, 12', 12" (+ un transducteur non visible sur la figure 4 puisqu'il se trouve solidarisé à la membrane du haut-parleur 13) comportant chacun une face inférieure 16 et une face supérieure 17,
- un haut-parleur 13,
- un élément adhésif 14 disposé sur le pourtour du haut-parleur 13,
- des pattes support 40 entre le haut-parleur 13 et les transducteurs 12, 12' et 12".

Dans ce mode de réalisation le transducteur ultrasonore non visible est fixé à la membrane du haut-parleur 13, la membrane est donc apte à entrainer le transducteur ultrasonore 12 en vibration basse fréquence, qui lui-même génère un coup basse fréquence reçu par le milieu viscoélastique. Un coup (ou vibration) reçu par le milieu viscoélastique 10 engendre la propagation d'une onde de cisaillement basse fréquence, qui se propage dans le milieu viscoélastique 10. Ce même transducteur ultrasonore non visible est apte à émettre et recevoir des signaux ultrasonores de façon à pouvoir déterminer la vitesse de déplacement de l'onde de cisaillement se propageant dans les tissus.

Il est entendu que le dispositif 400 peut comporter plus de quatre transducteurs ultrasonores.

Dans un mode de réalisation non limitatif, les pattes support 40 sont formées de plusieurs tiges 40 rigides dont une extrémité de chaque tige 40 est fixée à la partie fixe 20 du vibreur 13, et une autre extrémité est fixée à un des transducteurs 12, 12' et 12". On note que les tiges 40 peuvent être flexibles. Dans ce mode de réalisation, comme les pattes support 40 sont fixées à la partie fixe du vibreur 13 les transducteurs ultrasonores 12, 12' et 12" ne sont soumis à aucune vibration et peuvent être utilisés selon un mode échographie.

Dans une variante de réalisation non limitative non illustrée, les pattes support 40 sont formées de plusieurs tiges rigides dont une extrémité de chaque tige 40 est fixée à la partie mobile du vibreur 13, et une autre extrémité est fixée à un des transducteurs 12, 12' et 12". Dans ce mode de réalisation, comme les pattes support 40 sont fixées à la partie mobile du vibreur 13 les transducteurs ultrasonores 12, 12' et 12" sont, tout comme le vibreur directement fixé à la membrane du vibreur 18, soumis à un mouvement de vibration lorsque la partie mobile du vibreur 13 émet un ou plusieurs coup(s) basse fréquence.

Grâce à l'élément adhésif 14, et aux pattes support 40, l'ensemble haut-parleur 13 / transducteurs ultrasonores, est maintenu dans une position stable vis-à-vis du milieu viscoélastique, telle que les faces inférieures des transducteurs ultrasonores soient au contact de la surface 11 et s'étendent sensiblement parallèlement à la surface 11. Dans cette position stable, des mesures de paramètres ultrasonores peuvent être réalisées à différents endroits du milieu viscoélastique sans que l'opérateur ne touche le dispositif. Ainsi, les mesures obtenues ne dépendent pas de l'habilité de l'opérateur.

Il convient de noter que dans cet exemple non limitatif, les transducteurs ultrasonores périphériques sont positionnés sous forme de cercle dont le centre est le vibreur et un transducteur ultrasonore mais les transducteurs ultrasonores périphériques pourraient également être positionnés sous forme de barrette.

L'utilisation d'une pluralité de transducteurs ultrasonores permet d'effectuer une pluralité de mesures simultanément. Comme ces mesures sont effectuées dans les mêmes conditions, il est possible de les comparer.

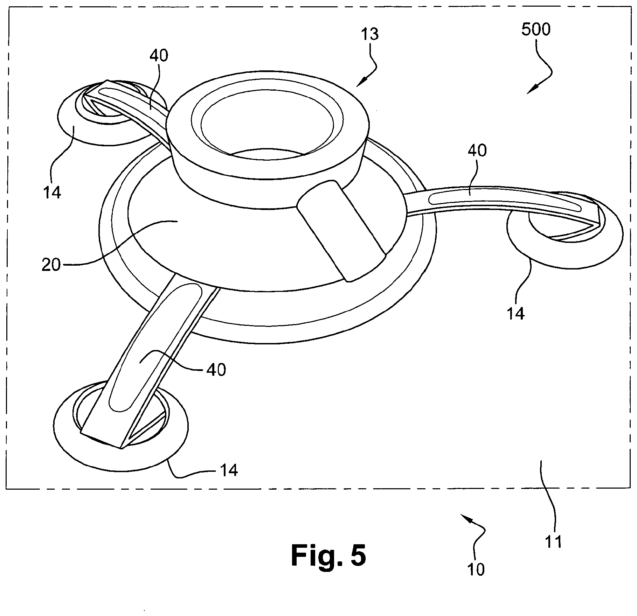

La figure 5 est une représentation schématique d'un cinquième mode de réalisation de l'invention. Le dispositif 500 comporte :
- un transducteur ultrasonore 12 (non illustré) en contact avec la surface 11 en regard du milieu viscoélastique 10,
- un haut-parleur 13 comportant trois pattes support 40, chacune des pattes support 40 comportant une première extrémité solidaire de la partie fixe 20 du vibreur 13 et une deuxième extrémité configurée pour être mise en appui sur la surface 11 en regard du milieu viscoelastique 10,
- un élément adhésif 14 est fixé au niveau de la deuxième extrémité de chacune des pattes support 40, chacun des éléments adhésif présentant une extrémité libre configurée pour être fixée par adhésion sur la surface 11 en regard du milieu viscoélastique 10 et maintenir la face d'émission et de réception du transducteur ultrasonore en regard de la surface 11.

D'une manière générale, les éléments adhésifs (ou moyens de maintien) sont adaptés pour maintenir la face d'émission et de réception 16, 16', 16" du (des) transducteur(s) 12, 12', 12" en regard d'une surface 11 en regard du milieu viscoélastique 10. En d'autres termes, les éléments adhésifs permettent de rendre solidaire le(s) transducteur(s) 12, 12', 12", et par voie de conséquence le vibreur 13 qui est solidaire du (des) transducteur(s) 12, 12', 12", de la surface 11 (par exemple l'épiderme) en regard du milieu viscoélastique 10 (par exemple le tissu adipeux situé sous l'épiderme) dont on souhaite déterminer les propriétés viscoélastiques. Ainsi, il est certain que les mesures obtenues via le dispositif ne sont pas opérateur-dépendant et les résultats obtenus seront les mêmes quelque soit l'opérateur. En effet, le transducteur ne sera pas incliné ni même déplacé au cours de la mesure.

En outre, le dispositif selon l'invention respecte les normes d'hygiène puisque les éléments adhésifs peuvent être changés après chaque utilisation, par exemple lorsque l'élément adhésif est un ruban autocollant de type médical.

Ce type d'élément adhésif est bien évidement non douloureux pour le patient.

Il convient par ailleurs de noter que la surface du ventre des patients obèses morbides présente une forme variable et une consistance variable (peau ferme et ventre rebondi ou ventre mou avec bourrelets). Le dispositif peut facilement être positionné sur de tels surface non plane.

Bien entendu, les modes de réalisation décrits sont donnés à titre d'exemple. Il est entendu que l'homme du métier est à même de réaliser différentes variations du dispositif pour la mesure de l'élasticité d'un milieu viscoélastique, en particulier concernant la disposition, le nombre ou l'agencement du ou des transducteur(s) ultrasonore(s) ou la forme des éléments adhésifs entre la cloche et le haut-parleur.

## Revendications

1. Dispositif (100, 200, 300, 400, 500) pour la mesure d'un paramètre ultrasonore ou biomécanique d'un milieu viscoélastique (10), ledit dispositif (100, 200, 300, 400, 500) comprenant au moins un transducteur ultrasonore (12, 12', 12"), ledit dispositif (100, 200, 300, 400, 500) étant **caractérisé en ce qu'**il comprend :
- au moins un vibreur (13) présentant une partie fixe (20) et une partie mobile (18), ledit transducteur ultrasonore (12, 12', 12") étant solidaire de ladite partie mobile (18) dudit au moins un vibreur (13) ;
- au moins un élément adhésif (14) solidaire du vibreur (13), ledit élément adhésif (14) étant configuré pour être fixé par adhésion sur une surface (11) en regard du milieu viscoélastique (10) et maintenir la face d'émission et de réception (16, 16', 16") du transducteur ultrasonore (12, 12', 12") en regard de la surface (11).

2. Dispositif (100, 200, 300, 400, 500) selon la revendication 1 précédente, **caractérisé en ce que** le au moins un vibreur (13) est un haut-parleur apte à vibrer.

3. Dispositif (100, 200, 300, 400, 500) selon l'une quelconque des revendications 1 ou 2 précédente, **caractérisé en ce que** l'élément adhésif (14) est fixé sur la partie fixe (18) du vibreur (13) et présente une extrémité libre adhésive configurée pour être fixée par adhésion sur la surface (11) en regard du milieu viscoélastique (10) et maintenir la face d'émission et de réception (16, 16', 16") du transducteur ultrasonore (12, 12', 12") en regard de la surface (11).

4. Dispositif (300) selon la revendication 3 précédente **caractérisé en ce que** l'extrémité libre adhésive est située sur le pourtour périphérique du vibreur (13).

5. Dispositif (500) selon l'une des revendications 1 ou 2 précédente **caractérisé en ce que** :
• le vibreur (13) comporte au moins deux pattes support (40), chacune desdites deux pattes support (40) comportant une première extrémité solidaire de la partie fixe (20) du vibreur (13) et une deuxième extrémité configurée pour être mise en appui sur la surface (11) en regard du milieu viscoelastique (10), et **en ce que**
• un élément adhésif (14) est fixé sur chacune des au moins deux pattes support (40), chaque élément adhésif (14) présentant une extrémité libre configurée pour être fixée par adhésion sur la surface (11) en regard du milieu viscoélastique (10) et maintenir la face d'émission et de réception (16, 16', 16") du transducteur ultrasonore (12, 12', 12") en regard de la surface (11).

6. Dispositif (400) selon l'une des revendications 1 ou 2 précédente **caractérisé en ce que** :
• le vibreur (13) comporte au moins deux pattes support (40), chacune desdites deux pattes support (40) comportant une première extrémité solidaire de la partie mobile (18) du vibreur (13) et une deuxième extrémité solidaire d'un transducteur ultrasonore (12, 12', 12"), et **en ce que**
• un élément adhésif (14) est fixé sur la partie fixe (20) du vibreur (13) et présente une extrémité libre adhésive située à la périphérie du vibreur (13) et configurée pour être fixée par adhésion sur la surface (11) en regard du milieu viscoélastique (10) et maintenir la face d'émission et de réception (16, 16', 16") des transducteurs ultrasonore (12, 12', 12") en regard de la surface (11).

7. Dispositif (400) selon l'une des revendications 1 ou 2 précédente **caractérisé en ce que** :
• le vibreur (13) comporte au moins deux pattes support (40), chacune desdites deux pattes support (40) comportant une première extrémité solidaire de la partie fixe (20) du vibreur (13) et une deuxième extrémité solidaire d'un transducteur ultrasonore (12, 12', 12"), et **en ce que**
• un élément adhésif (14) est fixé sur la partie fixe (20) du vibreur (13) et présente une extrémité libre adhésive située à la périphérie du vibreur (13) et configurée pour être fixée par adhésion sur la surface (11) en regard du milieu viscoélastique (10) et maintenir la face d'émission et de réception (16, 16', 16") des transducteurs ultrasonore (12, 12', 12") en regard de la surface (11).

8. Dispositif (200) selon l'une des revendications précédentes, **caractérisé en ce que** ledit dispositif comporte une membrane (22) apte à laisser passer des ultrasons sans les modifier, ladite membrane (22) étant insérée entre la face d'émission et de réception (16) du au moins un transducteur ultrasonore (12) et la surface (11) en regard du milieu viscoélastique (10).

9. Dispositif (100) selon l'une des revendications 1 ou 2 **caractérisé en ce qu'**il comporte des moyens de liaison (15) entre l'élément adhésif (14) et le vibreur (13).

10. Dispositif (400) selon l'une des revendications précédentes, **caractérisé en ce qu'**il comporte plusieurs transducteurs ultrasonores (12, 12', 12").

11. Dispositif (400) selon la revendication 10, **caractérisé en ce que** les transducteurs ultrasonores (12, 12', 12") sont disposés sur un cercle dont le centre est le vibreur (13).

12. Dispositif (100, 200, 300, 400, 500) selon l'une des revendications précédentes, **caractérisé en ce que** la fréquence centrale de vibration du vibreur (13) est comprise entre 20 et 1500 Hertz.

13. Dispositif (100, 200, 300, 400, 500) selon l'une des revendications précédentes, **caractérisé en ce que** la fréquence centrale du transducteur ultrasonore (12, 12', 12") est comprise entre 0,5 et 40 MegaHertz.

## Patentansprüche

1. Vorrichtung (100, 200, 300, 400, 500) für die Messung eines Ultraschall- oder biomechanischen Parameters eines viskoelastischen Milieus (10), wobei die genannte Vorrichtung (100, 200, 300, 400, 500) wenigstens einen Ultraschall-Signalwandler (12, 12', 12") umfasst, wobei die genannte Vorrichtung (100, 200, 300, 400, 500) **dadurch gekennzeichnet ist, dass** sie umfasst:
- wenigstens einen Vibrator (13), der einen festen Teil (20) und einen mobilen Teil (18) aufweist, wobei der genannte Ultraschall-Signalwandler (12, 12', 12") mit dem genannten mobilen Teil (18) des genannten wenigstens einen Vibrators (13) fest verbunden ist;
- wenigstens ein anhaftendes Element (14), das fest mit dem Vibrator (13) verbunden ist, wobei das genannte anhaftende Element (14) ausgestaltet ist, um per Anhaften auf einer Oberfläche (11) gegenüber dem viskoelastischen Milieu (10) befestigt zu sein und die Sende- und Empfangsseite (16, 16', 16") des Ultraschall-Signalwandlers (12, 12', 12") gegenüber der Oberfläche (11) zu halten.

2. Vorrichtung (100, 200, 300, 400, 500) gemäß dem voranstehenden Anspruch 1, **dadurch gekennzeichnet, dass** der wenigstens eine Vibrator (13) ein zum Vibrieren geeigneter Lautsprecher ist.

3. Vorrichtung (100, 200, 300, 400, 500) gemäß irgendeinem der voranstehenden Ansprüche 1 oder 2, **dadurch gekennzeichnet, dass** das anhaftende Element (14) auf dem festen Teil (18) des Vibrators (13) befestigt ist und ein anhaftendes freies Ende aufweist, das ausgestaltet ist, um per Anhaften auf der Oberfläche (11) gegenüber dem viskoelastischen Milieu (10) befestigt zu sein und die Sende- und Empfangsseite (16, 16', 16") des Ultraschall-Signalwandlers (12, 12', 12") gegenüber der Oberfläche (11) zu halten.

4. Vorrichtung (300) gemäß dem voranstehenden Anspruch 3, **dadurch gekennzeichnet, dass** das anhaftende freie Ende auf dem umlaufenden Umfang des Vibrators (13) angeordnet ist.

5. Vorrichtung (500) gemäß einem der voranstehenden Ansprüche 1 oder 2, **dadurch gekennzeichnet, dass**:
• der Vibrator (13) wenigstens zwei Trägerklauen (40) umfasst, wobei jede der genannten zwei Trägerklauen (40) ein erstes Ende, das mit dem festen Teil (20) des Vibrators (13) fest verbunden ist, und ein zweites Ende, das ausgestaltet ist, um auf der Oberfläche (11) gegenüber dem viskoelastischen Element (10) zum Aufstützen zu kommen, umfasst, und dass
• ein anhaftendes Element (14) auf jeder der wenigstens zwei Trägerklauen (40) befestigt ist, wobei jedes anhaftende Element (14) ein freies Ende aufweist, das ausgestaltet ist, um per Anhaften auf der Oberfläche (11) gegenüber dem viskoelastischen Milieu (10) befestigt zu sein und die Sende- und Empfangsseite (16, 16', 16") des Ultraschall-Signalwandlers (12, 12', 12") gegenüber der Oberfläche (11) zu halten.

6. Vorrichtung (400) gemäß einem der voranstehenden Ansprüche 1 oder 2, **dadurch gekennzeichnet, dass**:
• der Vibrator (13) wenigstens zwei Trägerklauen (40) umfasst, von denen jede der genannten zwei Trägerklauen (40) ein erstes Ende, das mit dem mobilen Teil (18) des Vibrators (13) fest verbunden ist, und ein zweites Ende, das mit einem Ultraschall-Signalwandler (12, 12', 12") fest verbunden ist, umfasst, und dass
• ein anhaftendes Element (14) auf dem festen Teil (20) des Vibrators (13) befestigt ist und ein anhaftendes freies Ende aufweist, das am Umfang des Vibrators (13) angeordnet und ausgestaltet ist, um per Anhaften auf der Oberfläche (11) gegenüber dem viskoelastischen Milieu (10) befestigt zu sein und die Sende- und Empfangsseite (16, 16', 16") der Ultraschall-Signalwandler (12, 12', 12") gegenüber der Oberfläche (11) zu halten.

7. Vorrichtung (400) gemäß einem der voranstehenden Ansprüche 1 oder 2, **dadurch gekennzeichnet, dass**:
• der Vibrator (13) wenigstens zwei Trägerklauen (40) umfasst, wobei jede der genannten zwei Trägerklauen (40) ein erstes Ende, das mit dem festen Teil (20) des Vibrators (13) fest verbunden ist, und ein zweites Ende, das fest mit einem Ultraschall-Signalwandler (12, 12', 12") fest verbunden ist, umfasst, und **dadurch gekennzeichnet, dass**
• ein anhaftendes Element (14) an dem festen Teil (20) des Vibrators (13) befestigt ist und ein anhaftendes freies Ende aufweist, das am Umfang des Vibrators (13) angeordnet und ausgestaltet ist, um per Anhaften auf der Oberfläche (11) gegenüber dem viskoelastischen Milieu (10) befestigt zu sein und die Sende- und Empfangsseite (16, 16', 16") der Ultraschall-Signalwandler (12, 12', 12") gegenüber der Oberfläche (11) zu halten.

8. Vorrichtung (200) gemäß einem der voranstehenden Ansprüche, **dadurch gekennzeichnet, dass** die genannte Vorrichtung eine Membran (22) umfasst, die geeignet ist, Ultraschall durchzulassen, ohne ihn zu verändern, wobei die genannte Membran (22) zwischen der Sende- und Empfangsseite (16) des wenigstens einen Ultraschall-Signalwandlers (12) und der Oberfläche (11) gegenüber dem viskoelastischen Milieu (10) eingefügt zu sein.

9. Vorrichtung (100) gemäß einem der Ansprüche 1 oder 2, **dadurch gekennzeichnet, dass** sie Verbindungsmittel (15) zwischen dem anhaftenden Element (14) und dem Vibrator (13) umfasst.

10. Vorrichtung (400) gemäß einem der voranstehenden Ansprüche, **dadurch gekennzeichnet, dass** sie mehrere Ultraschall-Signalwandler (12, 12', 12") umfasst.

11. Vorrichtung (400) gemäß Anspruch 10, **dadurch gekennzeichnet, dass** die Ultraschall-Signalwandler (12, 12', 12") auf einem Kreis angeordnet sind, deren Zentrum der Vibrator (13) ist.

12. Vorrichtung (100, 200, 300, 400, 500) gemäß einem der voranstehenden Ansprüche, **dadurch gekennzeichnet, dass** die zentrale Vibrationsfrequenz des Vibrators (13) zwischen 20 und 1500 Hertz inbegriffen ist.

13. Vorrichtung (100, 200, 300, 400, 500) gemäß einem der voranstehenden Ansprüche, **dadurch gekennzeichnet, dass** die zentrale Frequenz des Ultraschall-Signalwandlers (12, 12', 12") zwischen 0,5 und 40 Megahertz inbegriffen ist.

## Claims

1. Device (100, 200, 300, 400, 500) for measuring an ultrasonic or biomechanical parameter of a viscoelastic medium (10), said device (100, 200, 300, 400, 500) comprising at least one ultrasound transducer (12, 12', 12"), said device (100, 200, 300, 400, 500) being **characterised in that** it comprises:
- at least one vibrator (13) with a fixed part (20) and a moving part (18), said ultrasound transducer (12, 12', 12") being fixed to said moving part (18) of said at least one vibrator (13);
- at least one adhesive element (14) fixed to the vibrator (13), said adhesive element (14) being configured so that it is fixed by adhesion onto a surface (11) facing the viscoelastic medium (10) and holds the emission and reception face (16, 16', 16") of the ultrasound transducer (12, 12', 12") facing the surface (11).

2. Device (100, 200, 300, 400, 500) according to previous claim 1, **characterised in that** the at least one vibrator (13) is a loudspeaker capable of vibrating.

3. Device (100, 200, 300, 400, 500) according to either of the previous claims 1 or 2, **characterised in that** the adhesive element (14) is fixed onto the fixed part (18) of the vibrator (13) and it has a free adhesive end configured to be fixed by adhesion onto the surface (11) facing the viscoelastic medium (10) and hold the emission and reception face (16, 16', 16") of the ultrasound transducer (12, 12', 12") facing the surface (11).

4. Device (300) according to previous claim 3, **characterised in that** the adhesive free end is located on the peripheral contour of the vibrator (13).

5. Device (500) according to either of the previous claims 1 or 2, **characterised in that**:
• the vibrator (13) comprises at least two support tabs (40), each of said two support tabs (40) comprising a first end fixed to the fixed part (20) of the vibrator (13) and a second end configured to press on the surface (11) facing the viscoelastic medium (10), and **in that**
• an adhesive element (14) is fixed onto each of the at least two support tabs (40), each adhesive element (14) having a free end configured to be fixed by adhesion onto the surface (11) facing the viscoelastic medium (10) and to hold the emission and reception face (16, 16', 16") of the ultrasound transducer (12, 12', 12") facing the surface (11).

6. Device (400) according to either of the previous claims 1 or 2, **characterised in that**:
• the vibrator (13) comprises at least two support tabs (40), each of said two support tabs (40) comprising a first end fixed to the moving part (18) of the vibrator (13) and a second end fixed to an ultrasound transducer (12, 12', 12"), and **in that**
• an adhesive element (14) is fixed onto the fixed part (20) of the vibrator (13) and has a free adhesive end located at the periphery of the vibrator (13) and configured so that it can be fixed by adhesion onto the surface (11) facing the viscoelastic medium (10) and hold the emission and reception face (16, 16', 16") of the ultrasound transducers (12, 12', 12") facing the surface (11).

7. Device (400) according to either of the previous claims 1 or 2, **characterised in that**:
• the vibrator (13) comprises at least two support tabs (40), each of said two support tabs (40) comprising a first end fixed to the fixed part (20) of the vibrator (13) and a second end fixed to an ultrasound transducer (12, 12', 12"), and **in that**
• an adhesive element (14) is fixed onto the fixed part (20) of the vibrator (13) and has a free adhesive end located at the periphery of the vibrator (13) and configured so that it can be fixed by adhesion onto the surface (11) facing the viscoelastic medium (10) and hold the emission and reception face (16, 16', 16") of the ultrasound transducers (12, 12', 12") facing the surface (11).

8. Device (200) according to one of the previous claims, **characterised in that** said device comprises a membrane (22) capable of allowing ultrasounds to pass through without changing them, said membrane (22) being inserted between the emission and reception face (16) of at least one ultrasound transducer (12) and the surface (11) facing the viscoelastic medium (10).

9. Device (100) according to either claim 1 or 2, **characterised in that** it comprises bonding means (15) between the adhesive element (14) and the vibrator (13).

10. Device (400) according to one of the previous claims, **characterised in that** it comprises several ultrasound transducers (12, 12', 12").

11. Device (400) according to claim 10, **characterised in that** the ultrasound transducers (12, 12', 12") are arranged on a circle, with the vibrator (13) at the centre of the circle.

12. Device (100, 200, 300, 400, 500) according to one of the previous claims, **characterised in that** the central vibration frequency of the vibrator (13) is between 20 and 1500 Hertz.

13. Device (100, 200, 300, 400, 500) according to one of the previous claims, **characterised in that** the central frequency of the ultrasound transducer (12, 12', 12") is between 0.5 and 40 MegaHertz.
